# EUROPEAN PATENT APPLICATION

(11) **EP 1 935 884 A2**
(43) Date of publication of application: **25.06.2008**
(21) Application number: 08000114.2
(22) Date of filing: 06.12.2005
(51) Int. Cl.: C07D 211/32

(54) **New crystalline forms of donepezil base**

(30) Priority: 08.12.2004 US 6827
(62) Divisional of application: 05111723.2
(71) Applicant: CHEMAGIS LTD., 51200 Bnei-Brak (IL)
(72) Inventor: Adin, Itai, 84684 Beer-Sheva (IL); Iustain, Carmen, 84750 Beer-Sheva (IL); Arad, Oded, 76303 Rehovot (IL); Kaspi, Joseph, 53201 Givatayim (IL)
(74) Representative: Becker Kurig Straus

(57) **Abstract**

Novel crystalline forms of Donepezil base, processes for producing same, pharmaceutical compositions containing same and methods of treatment utilizing same are disclosed.

## Description

### FIELD AND BACKGROUND OF THE INVENTION

The present invention is of novel crystalline forms of Donepezil base and processes for their preparation.

Donepezil, (1-benzyl-4-[(5,6-dimethoxy-1-indanon)-2-yl]methylpiperidine) is represented by the formula I below.

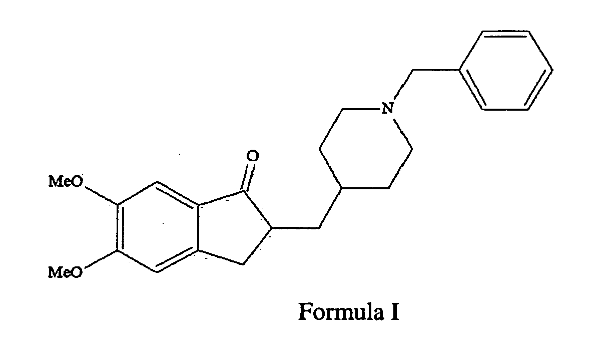

Donepezil is a piperidine derivative, which is chemically distinct from other agents in this class. It reversibly and noncompetitively inhibits centrally-active acetylcholinesterase. Donepezil was found to be effective in the treatment of dementia and Alzheimer's disease, which are related to cholinergic deficiency in the cortex and basal forebrain. Its cholinergic enhancement property is considered the reason for the improvement of the symptoms in the patients. The drug, formulated as 5 and 10 mg film coated tablets, is typically given once daily to the patients. Donepezil (as its hydrochloride salt) is marketed in the U.S. by Eisai America Inc. as Aricept^{®}.

The prophylactic and therapeutic activity of Donepezil hydrochloride for senile dementia, and in particular as a prophylactic and therapeutic agent for Alzheimer's disease, was first disclosed in U.S. Patent No. 4,895,841.

In a method described in U.S. Patent No. 4,895,841, a crude Donepezil product was purified by column chromatography, and the concentrated desired fractions were immediately converted to a hydrochloride salt. Accordingly, this patent fails to teach isolation of Donepezil in a solid or crystalline form.

Recently, three novel crystalline forms of Donepezil, referred to as forms A, B and C, were uncovered and characterized, as described in U.S. Patent No. 6,245,911. Powder diffraction patterns of these crystalline modifications of Donepezil taken from U.S. Patent No. 6,245,911 are given in Figures 1, 2 and 3.

U.S. patent Nos. 5,985,864 and 6,140,321 describe five different crystalline forms of Donepezil hydrochloride (including hydrates) as well as an amorphous Donepezil hydrochloride. The different crystalline forms of Donepezil hydrochloride, described in U.S. patent Nos. 5,985,864 and 6,140,321, may be prepared either by dissolution of the Donepezil hydrochloride salt in a polar solvent (such as methanol) under heating and addition of a less polar solvent (such as isopropyl ether) upon cooling followed by filtration of the separated crystals, or by dissolution of the Donepezil base form in a polar solvent (such as methanol) under heating and addition of alcoholic solution of concentrated hydrochloric acid followed by filtration of the separated crystals.

In U.S. Patent Application Publication No. 2004/0034057, a method is disclosed for industrial production of a polymorphic crystal form of Donepezil hydrochloride.

In recent years, solid-state properties of drugs received a great focus in the pharmaceutical industry, as a major contributing factor to both bio-availability and formulation characteristics. The ability of some substances to exist in more than one crystalline form, called polymorphism, was accredited as one of the most important solid-state property of drugs. While polymorphs have the same chemical composition, they differ in the packing and geometrical arrangement thereof, and exhibit different physical properties such as melting point, shape, color, density, hardness, deformability, stability, dissolution, and the like. Depending on their temperature-stability relationship, two polymorphs may be either monotropic or enantiotropic. For a monotropic system, the relative stability between the two solid phases remains unchanged as the temperature is changed. In contrast, in an enantiotropic system there exists a transition temperature at which the stability of the two phases reverse.

General background and theory of crystalline forms is found for example in "Effects of Polymorphism and Solid-State Solvation on Solubility and Dissolution Rate", in Polymorphism in Pharmaceutical Solids, edited by Harry G. Brittain, Drugs and the Pharmaceutical Sciences, Volume 95, MARCEL DEKKER, Inc.; "Effects of Pharmaceutical Processing on Drug Polymorphs and Solvates", in Polymorphism in Pharmaceutical Solids, edited by Harry G. Brittain; Drugs and the Pharmaceutical Sciences, Volume 95, MARCEL DEKKER, Inc.; "Theoretical approaches to physical transformations of active pharmaceutical ingredients during manufacturing process", Morris et al, Advanced drug delivery reviews, 48, 2001; and Theory and Origin of Polymorphism in "Polymorphism in Pharmaceutical Solids" (1999) ISBN: 8247-0237.

In some cases, different polymorphic forms of the same drug can exhibit very different solubility, and therefore different dissolution rates (release profile) *in-vivo.* This phenomenon may be used to create better drugs that dissolve either rapidly or very slowly, according to the specific needs of each formulation. However, any failure to predict the bioavailability of a drug may result in administration of either too small or too large undesired doses, which may be dangerous to patients and in extreme cases, lethal.

Other examples are known, where different crystalline forms behave differently during physical processing like milling and pressing. Many process-induced solid-solid transitions of substances are known, that lead to either other crystalline forms or an amorphous form of the substance. The solid-state experts are in a constant search for crystalline forms that can withstand physical stress and still retain their original properties.

Consequently, there is an ongoing search for new polymorphic forms of drugs, which may provide for improved performance thereof.

As described above, Donepezil hydrochloride exhibits polymorphic behavior. Its usage in pharmaceutical dosage forms is oftentimes limited by the hygroscopic and sticky characteristics thereof. In contrast, in U.S. Patent No. 6,245,911 it is sated that Donepezil base crystals have physical properties which allow it to occur in a non-sticky form, and which therefore enable excellent filtration after crystallization and facilitate facile recovery of the cake by scraping.

Other limitations in the production of Donepezil hydrochloride include its purification by crystallization. As taught in the art, the purification of Donepezil hydrochloride oftentimes require several crystallization cycles to yield a pharmaceutical purity, hence the process may suffer from relatively low yields.

Some of the methods used for obtaining the three novel crystalline forms of Donepezil base described in U.S. Patent No. 6,245,911 are cumbersome. Donepezil free base is in itself a pharmaceutical agent in addition to being a precursor for production of Donepezil hydrochloride.

There is a need for improved production processes for crystalline Donepezil base devoid of the limitations of the processes known in the art.

It is important to note that different crystalline forms of a pharmaceutically useful compound provide opportunities to improve the performance characteristics of a pharmaceutical product. Different crystalline forms enlarge the repertoire of materials that a formulation scientist has available for designing, for example, a pharmaceutical dosage form of a drug with a desired release profile, solubility characteristics or other desired characteristic. It is well known that new crystalline forms of known useful compounds are of utility.

There is thus a widely recognized need for, and it would be highly advantageous to have new and distinct crystalline forms of Donepezil.

### SUMMARY OF THE INVENTION

Thus, the present invention provides novel crystalline forms of Donepezil base, and processes for the preparation thereof.

The general techniques that may lead to the discovery of a novel crystalline form of a certain compound are well known to those who are skilled in the art. Such techniques may include crystallization, thermal treatment, and sublimation. Those who are skilled in the art appreciate that in a search for new polymorphic forms of a certain compound, anyone of these techniques is expected to fail. The search is an empirical exercise that involves series of trial and error experiments with different techniques and conditions. For these reasons, it is impossible to predict the experimental conditions that will produce a new Donepezil crystalline form. It is, however, possible to provide methods that have successfully and selectively produced Donepezil in one of these desired forms after conducting a series of trial and error experiments.

Herein novel crystalline forms of Donepezil base that are suitable for pharmaceutical use are presented.

Thus, according to one aspect of the present invention there is provided a crystalline Donepezil Form IV, which comprises at least one of the characteristics selected from the group consisting of: a powder X-ray diffraction pattern exhibiting peaks at diffraction angles of about 4.9, 9.8, 12.9, 13.6, 15.6, 16.3, 16.9, 17.9, 18.4, 18.8, 19.3, 19.7, 20.1, 20.7, 21.1, 22.5, 23.2, 23.6, 24.3, 24.7, 25.1, 25.5, 26.1, 26.6, 27.2, 27.5, 29.2, 29.6 and 30.5 ± 0.2 °2θ; and an infrared spectrum with characteristic absorption peaks at 3100-2700 cm⁻¹.

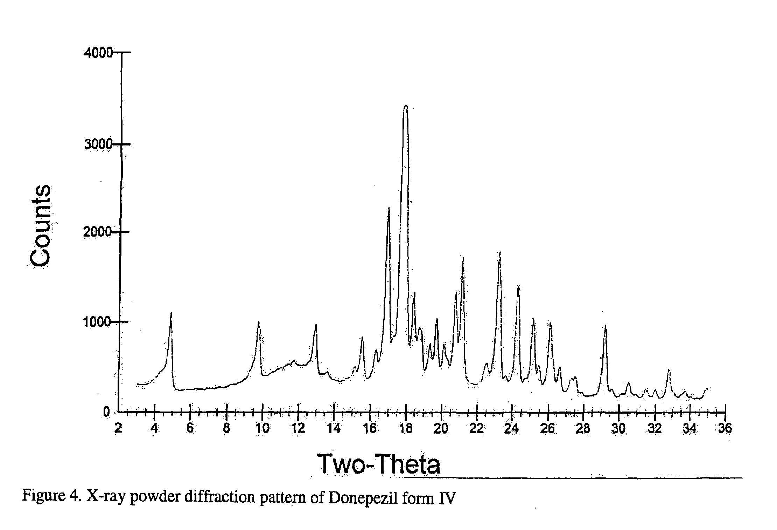

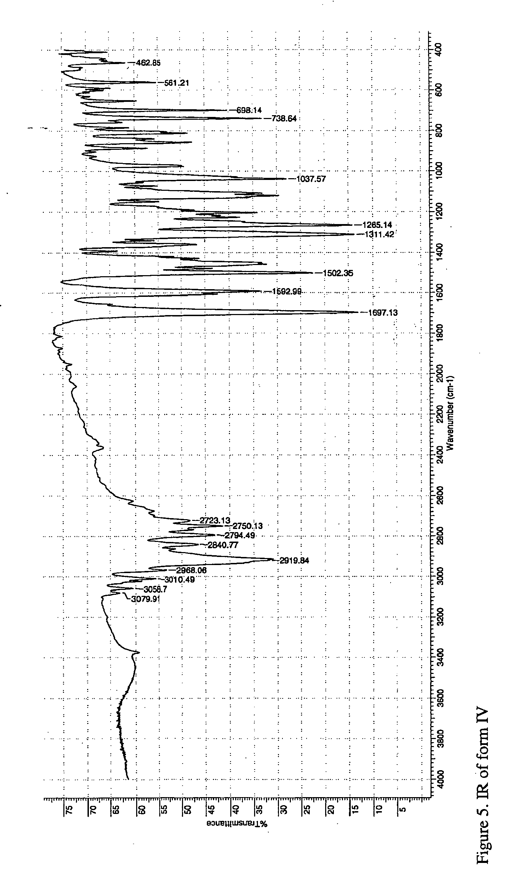

The powder X-ray diffraction pattern is substantially as depicted in Figure 4, whereby the infrared spectrum is substantially as depicted in Figure 5.

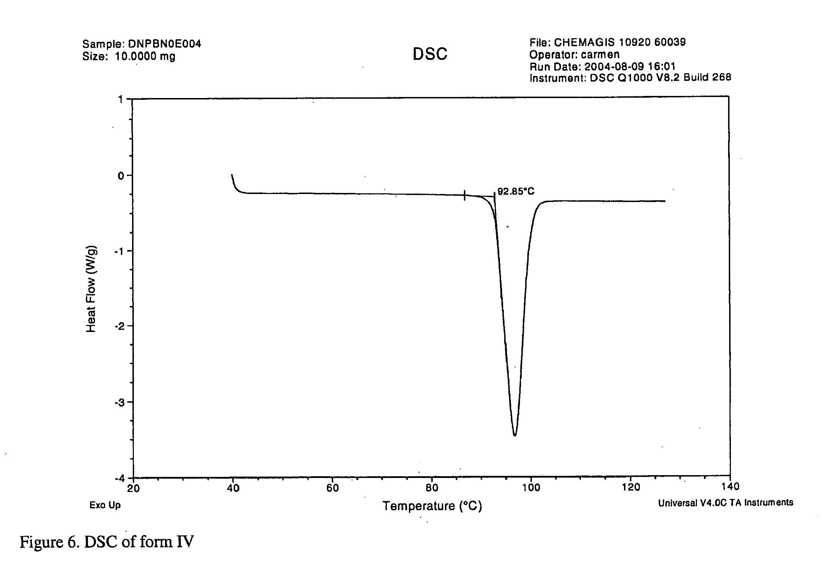

This crystalline Donepezil is preferably further characterized by a DSC curve substantially as depicted in Figure 6, and by a melting range of about 94-99 °C.

According to another aspect of the present invention there is provided a process of preparing the crystalline Donepezil Form IV described above. The process is effected by providing Donepezil; contacting the Donepezil with a solvent, the solvent including at least one solvent component selected from the group consisting of cyclohexane, nitroethane, xylene, dichloromethane, DMF, n-hexane, n-butanol, amyl alcohol, n-octane, ethanol, ethyl acetate, acetone, acetonitrile and butylamine, to thereby form a Donepezil solution; crystallizing the Donepezil in the solution, to thereby obtain the crystalline Donepezil Form IV; and isolating the crystalline Donepezil Form IV.

The solvent preferably includes at least 50 % by weight of the solvent component and more preferably substantially consists of the solvent component.

Thus, the solvent component can be cyclohexane, nitroethane, DMF, n-hexane, n-butanol, amyl alcohol, n-octane, ethanol, ethyl acetate, acetone, acetonitrile, butylamine, a mixture of xylene and dichloromethane wherein a ratio of the xylene to the dichloromethane is preferably between about 1: 10 and about 1:2 (v/v) and more preferably is about 1: 5 (v/v), or a mixture of toluene and isopropanol.

The concentration of the Donepezil in the Donepezil solution is preferably greater than 0.001 gram/ml.

Further preferably, during the dissolving the temperature of the solvent is raised to at least about 40 °C and the crystallizing includes allowing the Donepezil solution to cool down to a temperature lower than or equal to about 25 °C.

Further preferably, the isolating comprises separating the crystalline Donepezil Form IV from the solution.

Further preferably the process further comprises, subsequent to the separating, drying the crystalline Donepezil Form IV, whereby the drying is typically effected at room temperature.

Providing the Donepezil can be effected by converting Donepezil hydrochloride to the Donepezil, whereby the converting can be performed *in situ.*

In a preferred embodiment, this converting is effected by: providing a mixture of Donepezil hydrochloride, an organic solvent and water; contacting the mixture with an inorganic base, the inorganic base being capable of converting the Donepezil hydrochloride to the Donepezil, to thereby provide a mixture including a solution of the Donepezil in the organic solvent and an aqueous solution; separating the organic solution from the mixture; and distilling out at least a portion of the organic solvent.

Preferably, the organic solvent forms a part of the solvent component. An exemplary organic solvent is toluene, whereby the solvent component is a mixture of toluene and isopropanol.

An exemplary inorganic base is sodium carbonate.

According to still another aspect of the present invention there is provided a crystalline Donepezil Form V, which comprises at least one of the characteristics selected from the group consisting of: a powder X-ray diffraction pattern exhibiting peaks at diffraction angles of about 6.0, 6.3, 6.8, 6.9, 9.8, 15.0, 16.0, 16.7, 17.0, 17.9, 18.5, 18.7, 19.0, 19.3, 19.5, 19.8, 20.2, 20.6, 20.8, 21.4, 22.3, 22.5, 23.1, 23.7, 24.0, 24.2, 24.7, 24.8, 25,2, 25.6, 26.0, 26.5, 26.9, 27.0, 27.2, 27.7, 28.2, 28.5, 29.0, 29.5, 29.6, and 30.1 ±0.2 °2θ; and an infrared spectrum with characteristic absorption peaks at 3100-2700 cm⁻¹.

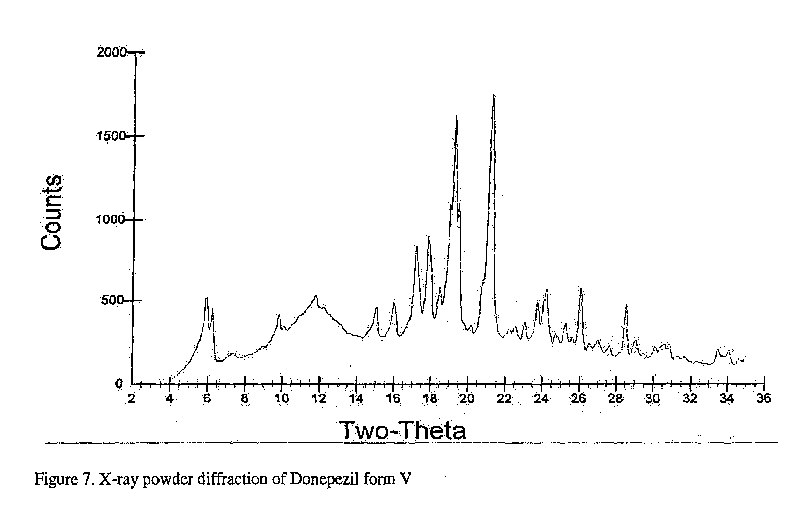

The powder X-ray diffraction pattern is substantially as depicted in Figure 7.

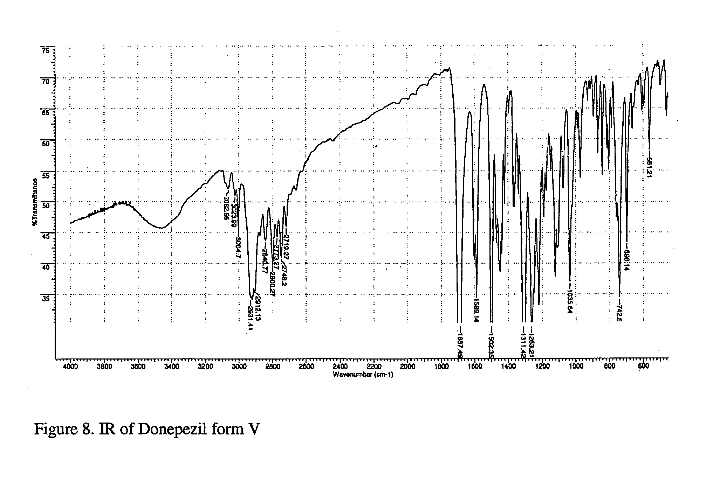

The infrared spectrum is substantially as depicted in Figure 8.

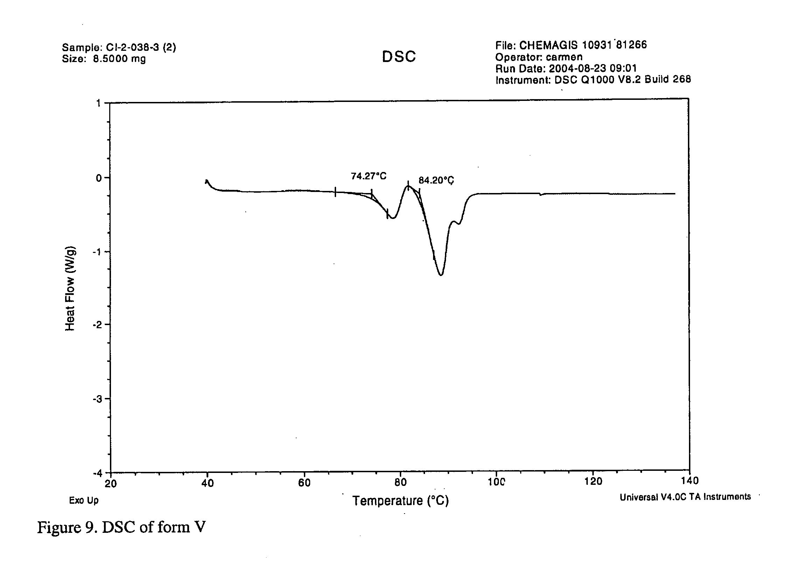

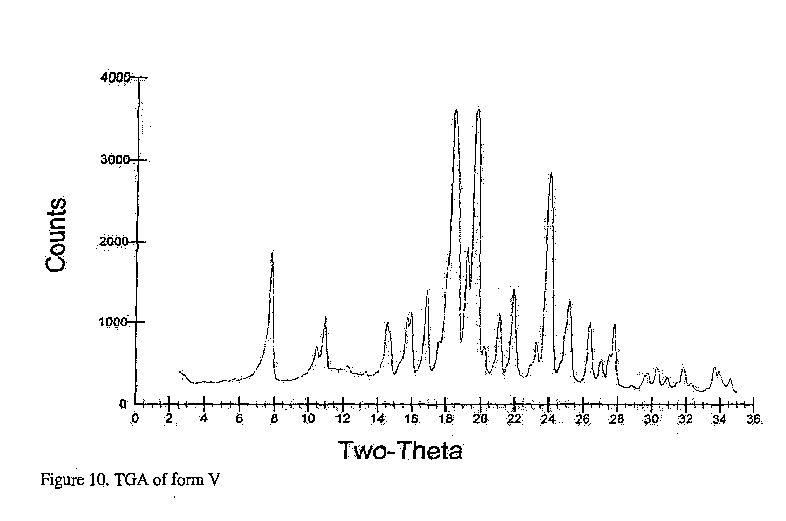

This crystalline Donepezil is preferably further characterized by a DSC curve substantially as depicted in Figure 9, and by a TGA curve substantially as depicted in Figure 10 and having a weight loss of 5-15 percent between 40-100 °C.

According to yet another aspect of the present invention there is provided a process of preparing crystalline Donepezil Form V. The process comprises: providing Donepezil, as described hereinabove; contacting the Donepezil with a solvent, as described hereinabove, whereby the solvent including chloroform as a solvent component, to thereby form a Donepezil solution; crystallizing the Donepezil in the solution, as described hereinabove, to thereby obtain the crystalline Donepezil Form V; and isolating the crystalline Donepezil Form V, as described hereinabove.

The concentration of Donepezil in the Donepezil solution is preferably greater than 0.001 gram/ml and more preferably is about 0.6 gram/ml.

During the dissolving the temperature of the solvent is preferably raised to at least about 50 °C.

The crystalline Donepezil Form V includes between 5 % and 15 % chloroform by weight.

According to an additional aspect of the present invention there is provided a crystalline Donepezil Form VI, which comprises at least one of the characteristics selected from the group consisting of: a powder X-ray diffraction pattern exhibiting peaks at diffraction angles of about 7.8, 10.5, 11.0, 12.3, 13.3, 14.5, 14.7, 15.7, 16.0, 16.9, 17.6, 18.0, 18.4, 18.6, 19.2, 19.7, 20.2, 21.1, 21.9, 22.9, 23.3, 24.0, 24.9, 25.2, 26.4, 27.1, 27.5, 27.8, 28.8, 29.6 and 30.3 ± 0.2 °2θ; and an infrared spectrum with υₘₐₓ at about 1678, 731 and 712 ± 4 cm⁻¹.

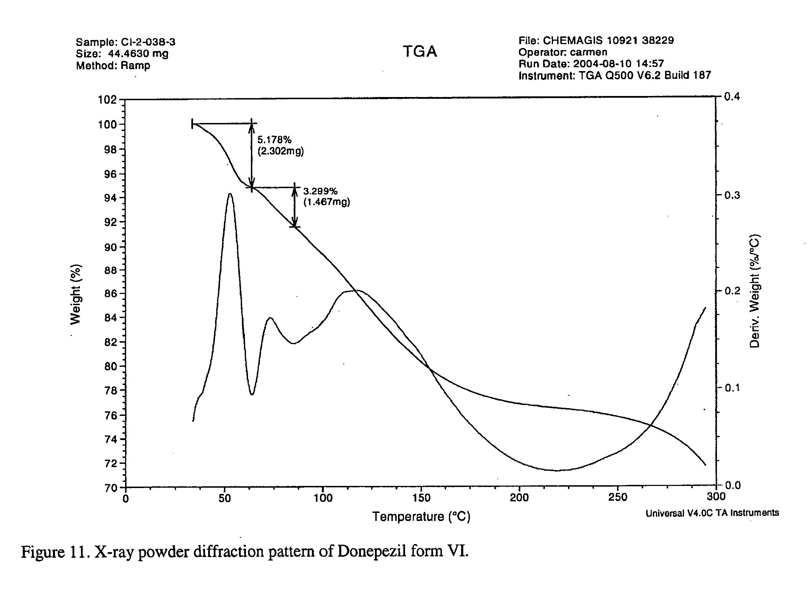

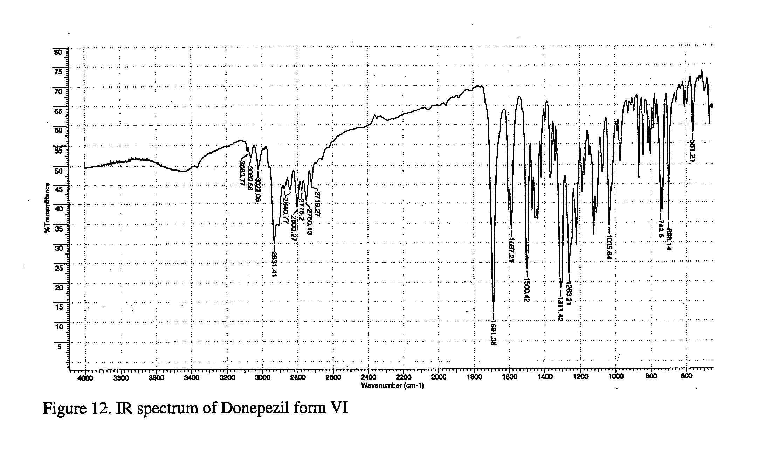

The powder X-ray diffraction pattern is substantially as depicted in Figure 11. The infrared spectrum is substantially as depicted in Figure 12.

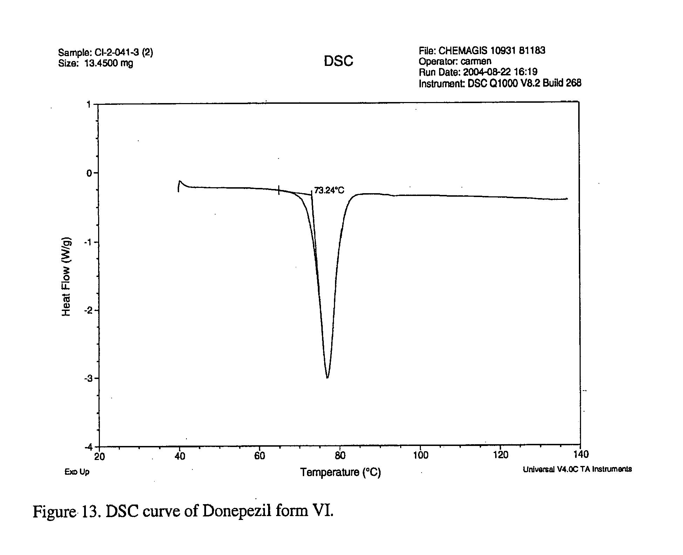

This crystalline Donepezil is preferably further characterized by a DSC curve substantially as depicted in Figure 13 and by a melting range of about 79-83.5 °C.

According to a further aspect of the present invention there is provided a process of preparing the crystalline Donepezil Form VI. The process comprises: providing Donepezil, as described hereinabove; contacting the Donepezil with a solvent, as described hereinabove, whereby the solvent including toluene as a solvent component, to thereby form a Donepezil solution; crystallizing the Donepezil in the solution, as described hereinabove, to thereby obtain the crystalline Donepezil Form VI; and isolating the crystalline Donepezil Form VI, as described hereinabove.

According to still a further aspect of the present invention there is provided a crystalline Donepezil Form VII, which comprises at least one of the characteristics selected from the group consisting of: a powder X-ray diffraction pattern exhibiting peaks at diffraction angles of about 4.9, 5.7, 7.7, 8.7, 9.8, 10.1, 10.5, 11.5, 12.3, 12.9, 13.3, 13.7, 13.9, 14.3, 14.7, 15.3, 15.6, 15.8, 16.3, 16.9, 17.2, 17.6, 17.9, 18.4, 18.8, 19.2, 19.7, 20.1, 20.3, 20.8, 21.1, 21.4, 21.6, 21.7, 22.0, 22.2, 22.5, 22.8, 23.2, 23.5, 23.7, 24.3, 24.6, 25.1, 25.5, 25.7, 26.1, 26.6, 27.3, 27.5, 29.2 and 30.5 ±0.2 °2θ; and an infrared spectrum with υₘₐₓ at about 3387, 1759 and 1734 ±4 cm⁻¹.

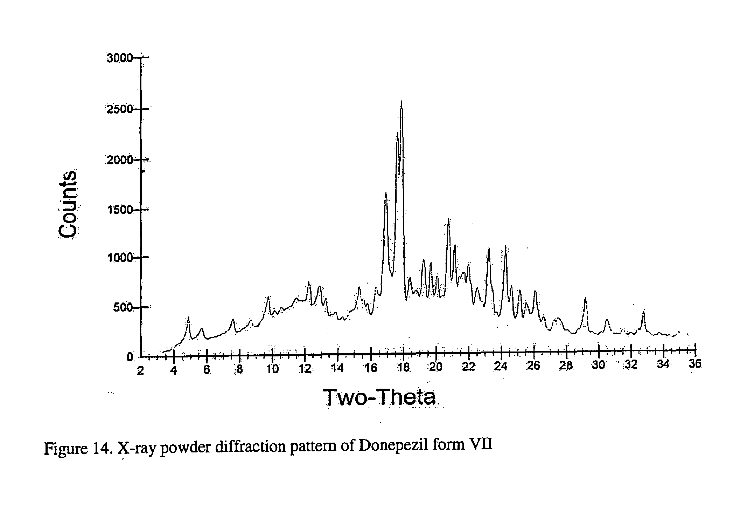

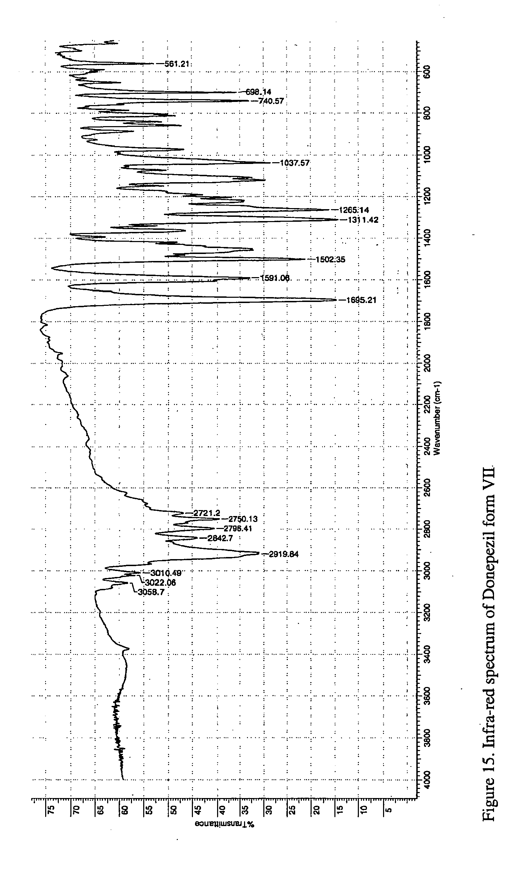

The powder X-ray diffraction pattern is substantially as depicted in Figure 14. The infrared spectrum is substantially as depicted in Figure 15.

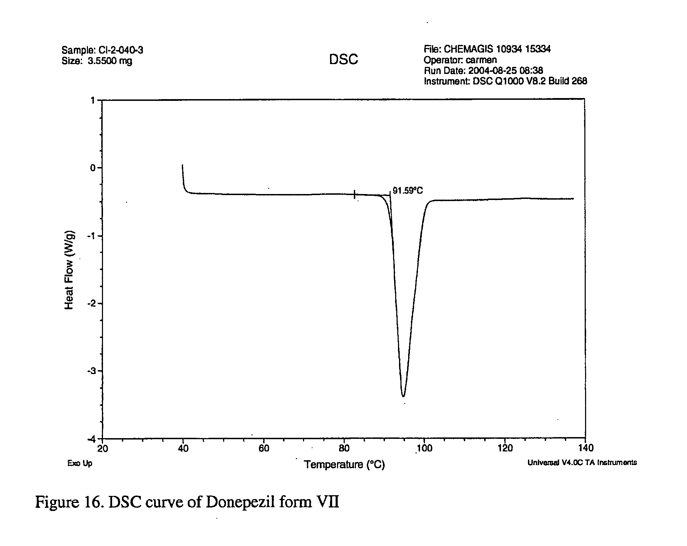

This crystalline Donepezil is preferably further characterized by a DSC curve substantially as depicted in Figure 16, and by a melting range of about 94-98 °C.

According to yet a further aspect of the present invention there is provided a process of preparing the crystalline Donepezil Form VII. The process comprises: providing Donepezil, as described hereinabove; contacting the Donepezil with a solvent, as described hereinabove, whereby the solvent including a mixture of methyl ethyl ketone and n-octane as a solvent component, to thereby form a Donepezil solution; crystallizing the Donepezil in the solution, as described hereinabove, to thereby obtain the crystalline Donepezil Form VII; and isolating the crystalline Donepezil Form VII, as described hereinabove.

The ratio of the methyl ethyl ketone to the n-octane is preferably between about 1: 20 and about 1:30 (v/v) and more preferably is about 1: 25 (v/v).

Contacting the Donepezil with the solvent is preferably effected by dissolving the Donepezil in the methyl ethyl ketone so as to obtain a Donepezil solution and thereafter adding the n-octane to the Donepezil solution.

The concentration of Donepezil in the Donepezil solution is preferably greater than 0.001 gram/ml of methyl ethyl ketone and more preferably is about 0.48 gram/ml methyl ethyl ketone.

According to an additional aspect of the present invention there is provided a process for preparing Donepezil hydrochloride, which comprises: providing at least one crystalline Donepezil selected from the group consisting of Donepezil Form IV, Donepezil Form V, Donepezil Form VI and Donepezil Form VII; and contacting the crystalline Donepezil with hydrochloric acid.

According to the teachings of the present invention there is also provided a pharmaceutical composition comprising at least one crystalline Donepezil selected from the group consisting of Donepezil Form IV, Donepezil Form V, Donepezil Form VI and Donepezil Form VII, described above, and a pharmaceutically acceptable carrier.

Preferably, a pharmaceutical composition of the present invention is fashioned in a delivery form selected from the group consisting of an aerosol delivery form, a bolus, a capsule, a delayed release capsule, a dissolvable powder, drops, a gel capsule, granules, an injectable solution, an ingestible solution, an inhalable solution, a pill, a suppository, a suspension, a syrup, a tablet, a tincture, a topical cream and a transdermal delivery form.

Most preferably, a pharmaceutical composition of the present invention is fashioned in a tablet delivery form. In an embodiment of the present invention each such tablet comprises between about 1 mg and about 50 mg Donepezil, preferably about 5 mg Donepezil or about 10 mg Donepezil.

In an embodiment of the present invention the pharmaceutical composition of the present invention is packaged in a packaging material and identified in print, in or on said packaging material, for use for a need selected from the group consisting of curing a condition, treating a condition, preventing a condition, treating symptoms of a condition, curing symptoms of a condition, ameliorating symptoms of a condition, treating effects of a condition, ameliorating effects of a condition, and preventing results of a condition in which treatment by Donepezil is beneficial. Such conditions include dementia and Alzheimer's disease.

According to the teachings of the present invention there is also provided a method of producing a Donepezil-containing medicament comprising: providing at least one Donepezil-containing component selected from the group consisting of Donepezil Form IV, Donepezil Form V, Donepezil Form VI and Donepezil Form VII; and combining the at least one Donepezil-containing component with a pharmaceutically acceptable carrier.

According to the teachings of the present invention there is also provided a method of treatment comprising administering a pharmaceutically effective amount of Donepezil to a mammal (preferably a human) in need thereof, wherein the Donepezil includes at least one crystalline Donepezil selected from the group consisting of Donepezil Form IV, Donepezil Form V, Donepezil Form VI and Donepezil Form VII.

According to a feature of the present invention the need arises from a medical condition selected from the group consisting of dementia and Alzheimer's disease and the need is selected from the group consisting of curing said condition, treating said condition, preventing said condition, treating symptoms of said condition, curing symptoms of said condition, ameliorating symptoms of said condition, treating effects of said condition, ameliorating effects of said condition, and preventing results of said condition.

In an embodiment of the method of the present invention, the administering is effected intestinally, intra-arterially, intradermally, intramedullarly, intramuscularly, intraocularly, intraperitoneally, intravenously, intraventricularly, rectally, subcutaneously, suppositorially, transmucosally, intranasally, parenterally, orally, topically, transdermally, bronchially, buccally, sublingually or mucosally, preferably orally.

In an embodiment of the present invention the administering is performed once daily.

In an embodiment of the present invention, the pharmaceutically effective amount is approximately between about 1 mg and about 50 mg Donepezil per dose, preferably about 5 mg Donepezil per dose or about 10 mg Donepezil per dose.

As used herein, the terms "comprising" and "including" or grammatical variants thereof are to be taken as specifying the stated features, integers, steps or components but do not preclude the addition of one or more additional features, integers, steps, components or groups thereof. These terms encompass the terms "consisting of" and "consisting essentially of".

The phrase "consisting essentially of" or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

The term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts. Implementation of the methods of the present invention involves performing or completing selected tasks or steps manually, automatically, or a combination thereof.

### BRIEF DESCRIPTION OF THE FIGURES

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

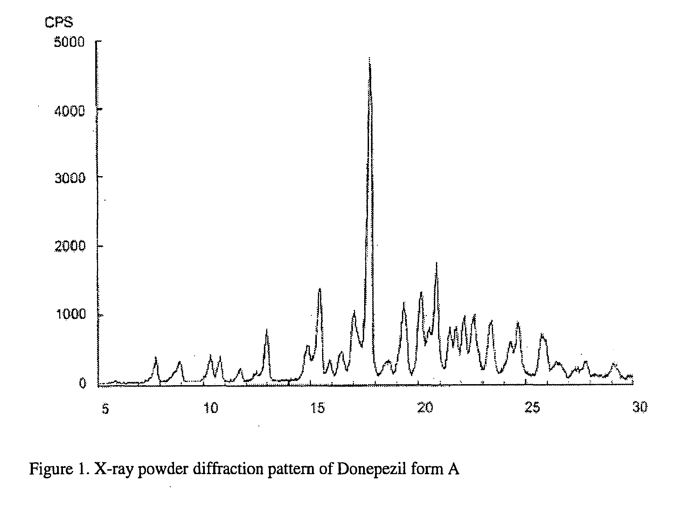

FIG. 1 presents an X-ray powder diffractogram of prior art Donepezil Form A;

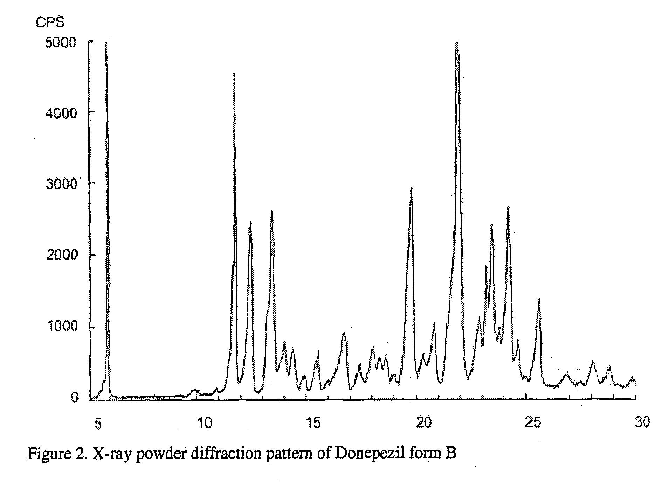

FIG. 2 presents an X-ray powder diffractogram of prior art Donepezil Form B;

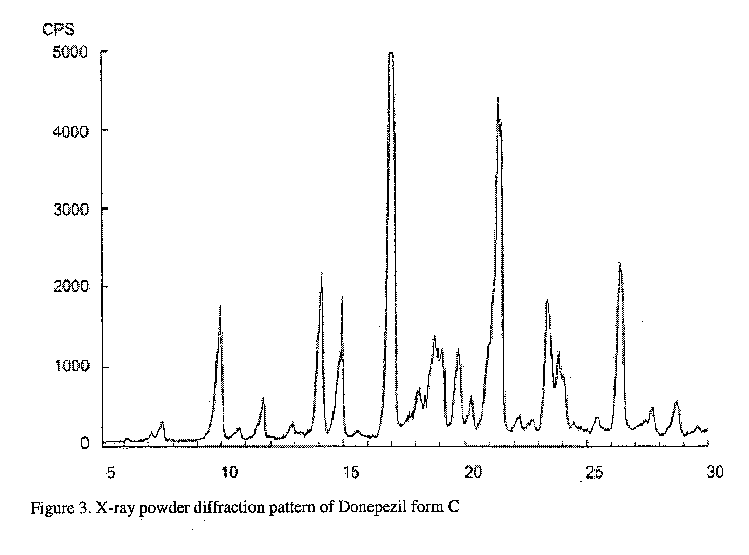

FIG. 3 presents an X-ray powder diffractogram of prior art Donepezil Form C;
FIG. 4 presents an X-ray powder diffractogram of Donepezil Form IV according to the present invention;
FIG. 5 presents an infrared spectrum of Donepezil Form IV according to the present invention;
FIG. 6 presents a differential scanning calorimetry curve of Donepezil Form IV according to the present invention;
FIG. 7 presents an X-ray powder diffractogram of Donepezil Form V according to the present invention;
FIG. 8 presents an infrared spectrum of Donepezil Form V according to the present invention;
FIG. 9 presents a differential scanning calorimetry curve of Donepezil Form V according to the present invention;
FIG. 10 presents the results of thermal gravimetric analysis of Donepezil Form V according to the present invention;
FIG. 11 presents an X-ray powder diffractogram of Donepezil Form VI according to the present invention;
FIG. 12 presents an infrared spectrum of Donepezil Form VI according to the present invention;
FIG. 13 presents a differential scanning calorimetry curve of Donepezil Form VI according to the present invention;
FIG. 14 presents an X-ray powder diffractogram of Donepezil Form VII according to the present invention;
FIG. 15 presents an infrared spectrum of Donepezil Form VII according to the present invention; and
FIG. 16 presents a differential scanning calorimetry curve of Donepezil Form VII according to the present invention.

### DESCRIPTION OF THE EMBODIMENTS

The novel crystalline forms of Donepezil of the present invention as well as processed for the preparation of the same are described hereinbelow. The experimental results are summarized in Table 1 (see the Examples section that follows).

Generally, the crystalline forms of the present invention are prepared by contacting Donepezil with a solvent, the solvent including at least one particular solvent component. One preferred method of contacting Donepezil with a solvent is by dissolving the Donepezil in the solvent (generally at elevated temperatures) and then crystallizing the dissolved Donepezil from the solvent using conventional techniques (e.g., slow or fast cooling, addition of a crystallization solvent), so as to form the desired crystalline form of Donepezil.

The nature of the particular solvent component determines which of the crystalline forms is produced. Generally a solvent includes at least 50 % by weight of an appropriate solvent component, preferably at least 70 % by weight of the solvent component, more preferably at least 80 % by weight of the solvent component, more preferably at least 90 % by weight of the solvent component or even substantially consists of the solvent component.

### Donepezil Form IV:

The present invention provides Donepezil form IV that produces a unique powder diffraction pattern, as presented in Figure 4). The reflections at 4.9, 9.8, 12.9, 16.9, 17.9, 20.7, 21.1, 23.2, 24.3, 25.1 and 26.1 ± 0.2 degrees 2θ are most characteristic of this form.

Donepezil form IV may be obtained by preparing a solution of Donepezil base in at least one suitable solvent, optionally at elevated temperature, cooling the solution thereby allow crystallization, collecting the crystals by filtration and drying.

The at least one suitable organic solvent is selected from the group consisting of cyclohexane, nitroethane, xylene, dichloromethane, DMF, n-hexane, n-butanol, amyl alcohol, n-octane, ethanol, ethyl acetate, acetone, acetonitrile, and butylamine or any mixtures thereof, as is detailed in the Examples section that follows.

The melting range of Donepezil Form IV is: 94-99 °C.

In Table 2, the position of peaks and position and the relative intensities of peaks of the powder X-ray diffractogram of Donepezil Form IV are presented.

Form IV produces a unique infrared (IR) spectrum, as presented in Figure 5. The area between 3100-2700 cm⁻¹ may be used as a characteristic fingerprint of this form.

The DSC curve of Donepezil form IV, presented in Figure 6, contains only the endothermic melting peak with onset around 92 ± 5 °C.

**Table 2 - Form IV - Powder X-ray diffraction peak positions and intensities**

| Relative Intensity (%) | Peak Position (2θ deg) | | Relative Intensity (%) | Peak Position (2θ deg) |
|---|---|---|---|---|
| 27 | 4.9 | | 8 | 22.5 |
| 23 | 9.8 | | 36 | 23.2 |
| 22 | 12.9 | | 4 | 23.6 |
| 4 | 13.6 | | 32 | 24.3 |
| 16 | 15.6 | | 4 | 24.7 |
| 7 | 16.3 | | 26 | 25.1 |
| 39 | 16.9 | | 10 | 25.5 |
| 100 | 17.9 | | 26 | 26.1 |
| 23 | 18.4 | | 13 | 26.6 |
| 12 | 18.8 | | 6 | 27.2 |
| 10 | 19.3 | | 9 | 27.5 |
| 21 | 19.7 | | 29 | 29.2 |
| 14 | 20.1 | | 5 | 29.6 |
| 31 | 20.7 | | 8 | 30.5 |
| 39 | 21.1 | | | |

### Donepezil Form V:

The present invention provides Donepezil form V which produces a unique powder X-ray diffraction pattern, as is presented in Figure 7. The reflections at 6.0, 6.3, 15.0, 16.0, 17.0, 17.9, 19.0, 19.3, 19.5 and 21.4 ± 0.2 degrees 20 are most characteristic of this form. Form V was measured with silicon grease as an adhesive. Additional broad peak is observed between 9-14 degrees 2θ.

Donepezil form V may be obtained by preparing a solution of Donepezil base in chloroform, optionally at elevated temperature, cooling the solution to thereby allow crystallization, collecting the crystals by filtration and drying, as is detailed in the Examples section that follows.

Donepezil form V is a solvate containing around 8.5 ± 3 % chloroform. As is shown in Figure 10, such weight loss was observed in TGA, between 40-90 °C. Apparently, this weight loss is part of a solid-solid transition of Donepezil form V to Donepezil form A. The weight loss and transition were also observed by DSC, presented in Figure 9. X-ray diffraction pattern of Donepezil dried form V at room temperature was similar to that of Donepezil form A.

In Table 3, the position of peaks and position and relative intensities of peaks of the powder X-ray diffractogram of Donepezil Form V are presented.

Additional weight loss at higher temperatures was observed in the TGA curve of this form, and was assigned to decomposition.

Donepezil form V produces a unique IR spectrum, as presented in Figure 8. The area between 3100-2700 cm⁻¹ can be used as a characteristic fingerprint of this form.

**Table 3 - Form V - Powder X-ray diffraction peak positions and intensities**

| Relative Intensity (%) | Peak Position (2θ deg) | | Relative Intensity (%) | Peak Position (2θ deg) |
|---|---|---|---|---|
| 30 | 6.0 | | 19 | 22.5 |
| 25 | 6.3 | | 21 | 23.1 |
| 10 | 6.8 | | 28 | 23.7 |
| 10 | 6.9 | | 28 | 24.0 |
| 26 | 9.8 | | 31 | 24.2 |
| 26 | 15.0 | | 17 | 24.7 |
| 28 | 16.0 | | 16 | 24.8 |
| 22 | 16.7 | | 20 | 25.2 |
| 47 | 17.0 | | 15 | 25.6 |
| 50 | 17.9 | | 32 | 26.0 |
| 33 | 18.5 | | 13 | 26.5 |
| 35 | 18.7 | | 11 | 26.9 |
| 62 | 19.0 | | 15 | 27.0 |
| 93 | 19.3 | | 12 | 27.2 |
| 62 | 19.5 | | 13 | 27.7 |
| 21 | 19.8 | | 10 | 28.2 |
| 20 | 20.2 | | 27 | 28.5 |
| 23 | 20.6 | | 15 | 29.0 |
| 35 | 20.8 | | 10 | 29.5 |
| 100 | 21.4 | | 9 | 29.6 |
| 19 | 22.3 | | 12 | 30.1 |

### Donepezil Form VI:

The present invention provides Donepezil form VI, which produces a unique powder X-ray diffraction pattern, as presented in Figure 11. The reflections at 7.8, 10.5, 11.0, 14.5, 14.7, 15.7, 16.0, 16.9, 18.4, 19.7, 21.1, 21.9, 24.0 and 25.2 ± 0.2 degrees 2θ are most characteristic of this form.

Donepezil form VI may be obtained by preparing a solution of Donepezil base in toluene, optionally at elevated temperature, cooling the solution to thereby allow crystallization, collecting the crystals by filtration and drying, as is detailed in the Examples section that follows.

The melting range of Donepezil Form VI is: 79-83.5 °C.

In Table 4, the position of peaks and position and relative intensities of peaks of the powder X-ray diffractogram of Donepezil Form VI are presented.

Infrared spectrum of Donepezil form VI is presented in Figure 12. The area between 3100-2700 cm⁻¹ can be used as a fingerprint for the identification of this form.

DSC curve of Donepezil form VI, presented in Figure 13, shows only the melting peak with onset around 73 ± 5 °C.

**Table 4 - Form VI - Powder X-ray diffraction peak positions and intensities**

| Relative Intensity (%) | Peak Position (2θ deg) | | Relative Intensity (%) | Peak Position (2θ deg) |
|---|---|---|---|---|
| 48 | 7.8 | | 16 | 20.2 |
| 15 | 10.5 | | 28 | 21.1 |
| 27 | 11.0 | | 36 | 21.9 |
| 6 | 12.3 | | 10 | 22.9 |
| 3 | 13.3 | | 21 | 23.3 |
| 23 | 14.5 | | 62 | 24.0 |
| 19 | 14.7 | | 23 | 24.9 |
| 25 | 15.7 | | 36 | 25.2 |
| 27 | 16.0 | | 30 | 26.4 |
| 33 | 16.9 | | 16 | 27.1 |
| 16 | 17.6 | | 18 | 27.5 |
| 38 | 18.0 | | 31 | 27.8 |
| 100 | 18.4 | | 3 | 28.8 |
| 56 | 18.6 | | 8 | 29.6 |
| 45 | 19.2 | | 15 | 30.3 |
| 96 | 19.7 | | | |

### Donepezil form VII:

The present invention provides Donepezil form VII, which crystallizes concomitantly with Donepezil form IV from a mixture of about 25:1 (V/V) n-octane:methylethyl ketone (MEK), as is detailed in the Examples section that follows.

The X-ray powder diffraction (XRPD) of Donepezil form IV was manually subtracted from the resulting XRPD of the mixture of polymorphs, presented in Figure 14, to produce the list of unique peaks of this form. The reflections at 5.8, 7.7, 8.8, 9.7, 10.2, 11.5, 11.6, 12.3, 13.2, 13.3, 14.2, 14.3, 15.4, 15.8, 16.3, 17.6, 19.3, 19.6, 21.1, 21.5, 21.7, 22.0, 23.2, 23.3, 24.3, 24.7, 25.3 and 25.6 ± 0.2 degrees 20 are characteristic of this form.

The melting range of a mixture of Donepezil Forms VII and IV is: 94-98 °C.

In Table 5, the position of peaks and position and relative intensities of peaks of the powder X-ray diffractogram of Donepezil Form VII are presented.

Infrared spectrum of Donepezil mixture of forms VII+IV is presented in Figure 15. It is very similar to the IR spectrum of form IV, and therefore does not point to the occurrence of a second phase.

The DSC curve of Donepezil form VII, presented in Figure 16, shows only an endothermic melting peak with onset around 92 ± 5 °C.

**Table 5 - Form VII - Powder X-ray diffraction peak positions and intensities**

| Relative Intensity (%) | Peak Position (2θ deg) | | Relative Intensity (%) | Peak Position (2θ deg) |
|---|---|---|---|---|
| 14 | 5.8 | | 100 | 17.6 |
| 8 | 7.7 | | 27 | 19.3 |
| 4 | 8.8 | | 24 | 19.6 |
| 20 | 9.7 | | 43 | 21.1 |
| 6 | 10.2 | | 18 | 21.5 |
| 6 | 11.5 | | 24 | 21.7 |
| 6 | 11.6 | | 31 | 22.0 |
| 25 | 12.3 | | 31 | 23.2 |
| 10 | 13.2 | | 63 | 23.3 |
| 8 | 13.3 | | 73 | 24.3 |
| 4 | 14.2 | | 27 | 24.7 |
| 4 | 14.3 | | 18 | 25.3 |
| 20 | 15.4 | | 20 | 25.6 |
| 8 | 15.8 | | | |
| 12 | 16.3 | | | |

Each of the Donepezil crystalline forms described above can be further reacted with hydrochloric acid, to thereby provide the corresponding crystalline Donepezil hydrochloride, as is detailed in the Examples section that follows.

In addition, each of the Donepezil crystalline forms described above can be produced using Donepezil hydrochloride as the starting material. As is exemplified in the Examples section that follows, Donepezil hydrochloride is converted to Donepezil base, which is thereafter crystallized *in situ,* without being separated from the reaction vessel with a selected solvent component, to thereby provide a crystalline Donepezil.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

### EXPERIMENTAL METHODS

The novel crystalline forms of Donepezil according to the present invention, have been characterized by powder X-ray diffraction, which produces a fingerprint of the particular crystalline form. Measurements of 2θ values typically are accurate to within ± 0.2 degrees 2θ.

X-ray diffraction data were acquired using a PHILIPS X-ray diffractometer model PW1050-70. System description: Kα1=1.54178Å, voltage 40kV, current 28 mA, diversion slit=1°, receiving slit=0.2mm, scattering slit=1° with a Graphite monochromator. Experiment parameters: pattern measured between 2θ=4° and 2θ=30° with 0.05° increments; count time was 0.5 second per increment

Wet Donepezil form V was measured using a silicon layer as an adhesive. This layer is observed as a hump between 9-14 degrees 2θ.

The novel crystalline forms of Donepezil have been further characterized by infrared spectroscopy. System description: Nicolet Furrier-transform infrared spectrometer model Avatar 360, with Omnic software version 5.2. All samples were run as KBr pellets. The current infrared measurements are accurate to within 4 cm⁻¹

The novel crystalline forms of Donepezil have been further characterized by differential scanning calorimetry (DSC), run on TA instruments model Q1000, with Universal software version 3.88. Samples were analyzed inside crimped 40 *µ*l Aluminum pans. Heating rate for all samples was 5 °C/min.

The novel crystalline forms of Donepezil have been further characterized by thermogravimetric analysis, run on TA instruments model Q500, with universal software version 3.88. Samples were run inside platinum baskets at heating rate of 5 °C/min.

### PREPARATION OF DONEPEZIL CRYSTALLINE FORMS

**Table 1- Summary of the experimental Results**

| Experiment No. | Donepezil form | Crystallization solvent or solvents | Experimental or production conditions |
|---|---|---|---|
| 1 | IV | cyclohexane | 1.2 g Donepezil dissolved in 80 ml solvent heating to reflux |
| 2 | IV | nitroethane | 2.3 g Donepezil dissolved in 3 ml solvent heating to reflux |
| 3 | IV | DMF | 1.7 g Donepezil dissolved in 25 ml solvent heating to 61°C |
| 4 | IV | n-hexane | 1.6 g Donepezil dissolved in 80 ml solvent heating to reflux |
| 5 | IV | n-butanol | 1.75 g Donepezil dissolved in 3 ml solvent heating to 60°C |
| 6 | IV | amyl alcohol | 1.6 g Donepezil dissolved in 3 ml solvent heating to 60°C |
| 7 | IV | n-octane | 1.0 g Donepezil dissolved in 20 ml solvent heating to 80°C |
| 8 | IV | ethanol | 1.0 g Donepezil dissolved in 2.5 ml solvent heating to reflux |
| 9 | IV | ethyl acetate | 1.2 g Donepezil dissolved in 2.5 ml solvent heating to reflux |
| 10 | IV | acetone | 1.5 g Donepezil dissolved in 2.5 ml solvent heating to reflux |
| 11 | IV | acetonitrile | 1.3 g Donepezil dissolved in 3 ml solvent heating to reflux |
| 12 | IV | n-butylamine | 1.5 g Donepezil dissolved in 2.5 ml solvent heating to 65°C |
| 13 | IV | xylene/dichloromethane | 1.5 g Donepezil dissolved in 5 ml xylene heating to 76°C, adding 15 ml xylene followed by 100 ml of dichloromethane at 65°C |
| 14 | IV | toluene/isopropanol | 15 Kg Donepezil HCl mixed with 75L each toluene and water heating to 60-65°C, separating, distilling out toluene and adding 75L isopropanol |
| 15 | V | chloroform | 1.8 g Donepezil dissolved in 3 ml solvent heating to reflux |
| 16 | VI | toluene | 1.5 g Donepezil dissolved in 2.5 ml solvent heating to reflux |
| 17 | VII | methyl ethyl ketone/ n-octane | 1.0 g Donepezil dissolved in 2.1 ml methyl ethyl ketone heating to reflux and adding 50 ml n-octane |
| 18 | IV | ethanol | 1.5 g Donepezil dissolved in 10 ml solvent heating to 35°C, adding HCl |

### EXAMPLE 1

### Preparation of Donepezil form IV

Donepezil (1.2 gram) was dissolved in cyclohexane (80 ml) in a 100 ml three-necked round bottom flask equipped with reflux condenser, thermometer and magnetic stirrer. The solution was heated using an oil bath to reflux, and was allowed to cool down to 25 °C. The resulting crystals (1.0 gram) were filtered off and left to dry in the hood.

### EXAMPLE 2

### Preparation of Donepezil form IV

Donepezil (2.3 grams) was dissolved in nitroethane (3 ml) in a 50 ml three necked round bottom flask equipped with reflux condenser, thermometer and magnetic stirrer. The solution was heated using an oil bath to reflux, and was allowed to cool down to 25 °C.

The resulting crystals (1.5 gram) were filtered and left to dry in the hood.

### EXAMPLE 3

### Preparation of Donepezil form IV

Donepezil (1.7 gram) was dissolved in dimethylformamide (DMF, 25 ml) in a 100 ml three necked round bottom flask equipped with reflux condenser, thermometer and magnetic stirrer. The solution was heated using an oil bath to 61 °C, and was allowed to cool down to 25 °C. The resulting crystals (1.5 gram) were filtered off and left to dry in the hood.

### EXAMPLE 4

### Preparation of Donepezil form IV

Donepezil (1.6 gram) was dissolved in n-hexane (80 ml) in a 100 ml three necked round bottom flask equipped with reflux condenser, thermometer and magnetic stirrer. The solution was heated using an oil bath to reflux, and was allowed to cool down to 25 °C. The resulting crystals (1.3 gram) were filtered off and left to dry in the hood.

### EXAMPLE 5

### Preparation of Donepezil form IV

Donepezil (1.75 gram) was dissolved in n-butanol (3 ml) in a 50 ml three necked round bottom flask equipped with a reflux condenser, thermometer and magnetic stirrer. The solution was heated using an oil bath to 60 °C, and was allowed to cool down to 25 °C. The resulting crystals (0.88 gram) were filtered off and left to dry in the hood.

### EXAMPLE 6

### Preparation of Donepezil form IV

Donepezil (1.6 gram) was dissolved in amyl alcohol (3 ml) in a 50 ml three-necked round bottom flask equipped with a reflux condenser, thermometer and magnetic stirrer. The solution was heated using an oil bath to 60 °C, and was allowed to cool down to 25 °C. The resulting crystals (1.3 gram) were filtered off and left to dry in the hood.

### EXAMPLE 7

### Preparation of Donepezil form IV

Donepezil (1.0 gram) was dissolved in n-octane (20 ml) in a 100 ml three-necked round bottom flask equipped with a reflux condenser, thermometer and magnetic stirrer. The solution was heated using an oil bath to 80 °C, and additional n-octane was added (5 ml), to initiate crystallization. The crystals dispersion was allowed to cool down to 25 °C. The resulting crystals (0.7 gram) were filtered off and left to dry in the hood.

### EXAMPLE 8

### Preparation of Donepezil form IV

Donepezil (1.0 gram) was dissolved in ethanol (2.5 ml) in a 50 ml three-necked round bottom flask equipped with a reflux condenser, thermometer and magnetic stirrer. The solution was heated using an oil bath to reflux, and was allowed to cool down to 25 °C. The resulting crystals (0.85 gram) were filtered off and left to dry in the hood.

### EXAMPLE 9

### Preparation of Donepezil form IV

Donepezil (1.2 gram) was dissolved in ethyl acetate (25 ml) in a 100 ml three-necked round bottom flask equipped with a reflux condenser, thermometer and magnetic stirrer. The solution was heated using an oil bath to reflux, and was allowed to cool down to 25 °C. The resulting crystals (1.2 gram) were filtered off and left to dry in the hood.

### EXAMPLE 10

### Preparation of Donepezil form IV

Donepezil (1.5 gram) was dissolved in acetone (2.5 ml) in a 50 ml three-necked round bottom flask equipped with a reflux condenser, thermometer and magnetic stirrer. The solution was heated using an oil bath to reflux, and was allowed to cool down to 25 °C. The resulting crystals (0.4 gram) were filtered off and left to dry in the hood.

### EXAMPLE 11

### Preparation of Donepezil form IV

Donepezil (1.3 gram) was dissolved acetonitrile (3 ml) in a 50 ml three-necked round bottom flask equipped with reflux condenser, thermometer and magnetic stirrer. The solution was heated using an oil bath to reflux, and was allowed to cool down to 25 °C. The resulting crystals (1.2 gram) were filtered off and left to dry in the hood.

### EXAMPLE 12

### Preparation of Donepezil form IV

Donepezil (1.5 gram) was dissolved in n-butylamine (2.5 ml) in a 50 ml three-necked round bottom flask equipped with a reflux condenser, thermometer and magnetic stirrer. The solution was heated using an oil bath to 65 °C, and was allowed to cool down to 25 °C. The wet resulting crystals (1.7 gram) were filtered off and left to dry in the hood.

### EXAMPLE 13

### Preparation of Donepezil form IV

Donepezil (1.5 gram) was dissolved in xylene (5 ml) in a 100 ml three-necked round bottom flask equipped with reflux condenser, thermometer and magnetic stirrer. The solution was heated using an oil bath to 76 °C. Additional xylene was added (15 ml). The solution temperature was lowered to 65 °C, and 100 ml methylene chloride were added. The resulting solution was allowed to cool down to 25 °C. The resulting crystals (0.64 gram) were filtered and left to dry in the hood.

### EXAMPLE 14

### Large scale preparation of Donepezil form IV from Donepezil HCl

Donepezil hydrochloride (15 Kg) was mixed with water (75 liters) and toluene (75 liters) in a jacketed 100 liter reactor, and stirred for 15 minutes. Sodium bicarbonate (3.8 kg) was added to the reactor and the solution was heated to 60-65 °C and left at that temperature during 30 minutes. The organic phase was separated from the aqueous layer, and toluene was distilled out (final temperature 70 °C). Isopropanol (75 liters) was added to the reactor, and the solution was heated to 60-65 °C until complete dissolution. The solution was cooled to 25 °C during 2 hours and left with stirring at that temperature during additional 2 hours. The temperature was further lowered to 10 °C and left with stirring at that temperature during 6 hours. The resulting crystals were filtered and washed with cold isopropanol, and dried to yield 10.3 Kg of Donepezil.

### EXAMPLE 15

### Preparation of Donepezil Form V

Donepezil (1.8 gram) was dissolved in chloroform (3 ml) in a 100 ml three necked round bottom flask equipped with a reflux condenser, thermometer and magnetic stirrer. The solution was heated using an oil bath to reflux, and was allowed to cool down to 25 °C. The resulting wet crystals (2.3 grams) were filtered off and left to dry in the hood.

### EXAMPLE 16

### Preparation of Donepezil Form VI

Donepezil (1.5 gram) was dissolved in toluene (2.5 ml) in a 100 ml three-necked round bottom flask equipped with reflux condenser, thermometer and magnetic stirrer. The solution was heated using an oil to reflux, and was allowed to cool down to 25 °C. The resulting crystals (1.5 gram) were filtered off and left to dry in the hood.

### EXAMPLE 17

### Preparation of Donepezil Form VII

Donepezil (1.0 gram) was dissolved in methyl ethyl ketone (2.1 ml) in a 100 ml three-necked round bottom flask equipped with reflux condenser, thermometer and magnetic stirrer. The solution was heated using an oil bath to reflux (Complete dissolution was observed at 40° C). n-Octane was added (50 ml), and the solution was allowed to cool down to 25 °C. The resulting crystals (0.64 gram) were filtered off and left to dry in the hood.

### EXAMPLE 18

### Preparation of Donepezil hydrochloride from Donepezil form IV

Donepezil (1.5 gram) was dissolved in ethanol (10 ml) in a 100 ml three-necked round bottom flask equipped with a reflux condenser, thermometer and magnetic stirrer. The solution was heated using an oil bath to 35 °C, and concentrated hydrochloric acid was added (0.45 gram) while maintaining the temperature as 35 °C. The heating was discontinued and thereafter the temperature was allowed to reach 25 °C while being mixed in order to initiate crystallization. Mixing was continued overnight at 10 °C. The resulting crystals (1.43 gram) were filtered off and left to dry in the hood.

This procedure is similarly performed with other polymorphic forms of Donepezil base described herein

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, the present invention is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. Crystalline Donepezil Form IV comprising at least one of the characteristics selected from the group consisting of:
a powder X-ray diffraction pattern exhibiting peaks at diffraction angles of about 4.9, 9.8, 12.9, 13.6, 15.6, 16.3, 16.9, 17.9, 18.4, 18.8, 19.3, 19.7, 20.1, 20.7, 21.1, 22.5, 23.2, 23.6, 24.3, 24.7, 25.1, 25.5, 26.1, 26.6, 27.2, 27.5, 29.2, 29.6 and 30.5 ± 0.2 °2θ ;and
an infrared spectrum with characteristic absorption peaks at 3100-2700 cm⁻¹.

2. The crystalline Donepezil of claim 1,
wherein said powder X-ray diffraction pattern is substantially as depicted in Figure 4; or
wherein said infrared spectrum is substantially as depicted in Figure 5; or
further **characterized by** a DSC curve substantially as depicted in Figure 6; or
further **characterized by** a melting range of about 94-99 °C.

3. A process of preparing crystalline Donepezil Form IV, the process comprising:
providing Donepezil;
contacting said Donepezil with a solvent, said solvent including at least one solvent component selected from the group consisting of cyclohexane, nitroethane, xylene, dichloromethane, DMF, n-hexane, n-butanol, amyl alcohol, n-octane, ethanol, ethyl acetate, acetone, acetonitrile and butylamine, to thereby form a Donepezil solution;
crystallizing said Donepezil in said solution, to thereby obtain the crystalline Donepezil Form IV; and
isolating the crystalline Donepezil Form IV.

4. The process of claim 3, wherein
said solvent includes at least 50 % by weight of said solvent component; or
said solvent substantially consists of said solvent component; or
said solvent component is cyclohexane; or
said solvent component is nitroethane; or
said solvent component is DMF; or
said solvent component is n-hexane; or
said solvent component is n-butanol; or
said solvent component is amyl alcohol; or
said solvent component is n-octane; or
said solvent component is ethanol; or
said solvent component is ethyl acetate; or
said solvent component is acetone; or
said solvent component is acetonitrile; or
said solvent component is butylamine; or
said solvent component is a mixture of toluene and isopropanol.

5. The process of claim 3, wherein said solvent component is a mixture of xylene and dichloromethane.

6. The process of claim 5, wherein a ratio of said xylene to said dichloromethane is between about 1: 10 and about 1:2 (v/v).

7. The process of claim 6, wherein a ratio of said xylene to said dichloromethane is about 1: 5 (v/v).

8. The process of claim 5, wherein said contacting said Donepezil with said solvent includes dissolving said Donepezil in said xylene so as to obtain a Donepezil solution and thereafter adding said dichloromethane to said Donepezil solution.

9. The process of claim 3, wherein said contacting said Donepezil with said solvent includes dissolving said Donepezil so as to obtain a Donepezil solution.

10. The process of claim 9, wherein
a concentration of said Donepezil in said Donepezil solution is greater than 0.001 gram/ml; or
during said dissolving the temperature of said solvent is raised to at least about 40 °C; or
and said crystallizing includes allowing said Donepezil solution to cool down to a temperature lower than or equal to about 25 °C.

11. The process of claim 3, wherein said isolating comprises separating said crystalline Donepezil Form IV from said solution.

12. The process of claim 11, further comprising, subsequent to said separating, drying said crystalline Donepezil Form IV.

13. The process of claim 12, wherein said drying is effected at room temperature.

14. The process of claim 3, wherein providing said Donepezil includes converting Donepezil hydrochloride to said Donepezil.

15. The process of claim 14, wherein said converting is performed in situ.

16. The process of claim 15, wherein said converting comprises:
providing a mixture of Donepezil hydrochloride, an organic solvent and water;
contacting said mixture with an inorganic base, said inorganic base being capable of
converting said Donepezil hydrochloride to said Donepezil, to thereby provide a mixture including a solution of said Donepezil in said organic solvent and an aqueous solution;
separating said organic solution from said mixture; and
distilling out at least a portion of said organic solvent.

17. The process of claim 16, wherein said organic solvent forms a part of said solvent component.

18. The process of claim 17, wherein
said organic solvent is toluene; or
said inorganic base is sodium carbonate; or
said solvent component is a mixture of toluene and isopropanol.

19. A process for preparing Donepezil hydrochloride comprising:
providing crystalline Donepezil Form IV; and
contacting said crystalline Donepezil with hydrochloric acid.
